# EUROPEAN PATENT APPLICATION

(11) **EP 4 209 500 A1**
(43) Date of publication of application: **12.07.2023**
(21) Application number: 21864594.3
(22) Date of filing: 26.08.2021
(51) Int. Cl.: C07K 5/10, C07K 7/06, A61K 8/64, A61Q 19/08, A61K 38/07, A61K 38/08, A61P 3/00

(54) **PEPTIDE, AND COSMETIC COMPOSITION AND PHARMACEUTICAL COMPOSITION COMPRISING SAME**

(30) Priority: 04.09.2020 KR 20200113278
(71) Applicant: Seoul National University Hospital, Seoul 03080 (KR)
(72) Inventor: CHUNG, Jin Ho, Seoul 04428 (KR); KIM, Eun Ju, Seoul 05217 (KR)
(74) Representative: dompatent von Kreisler Selting Werner - Partnerschaft von Patent- und Rechtsanwälten mbB
(86) International application number: PCT/KR2021/011446
(87) International publication number: WO 2022/050634

(57) **Abstract**

The present invention relates to a peptide as well as a cosmetic composition and a pharmaceutical composition including the peptide, which have excellent solubility and stability such that they are advantageous for formulation when manufacturing cosmetics or drugs containing the same, thereby improving skin wrinkles, sensitive skin and dermatitis, promoting hair growth, and enhancing drug efficacy for metabolic diseases.

## Description

### [Technical Field]

The present invention relates to a peptide, and a cosmetic composition and a pharmaceutical composition including the same.

### [Background Art]

Skin aging is often caused by external and internal processes associated with an increase in skin wrinkles, sagging and loosing. Because external aging is mainly caused by repeated exposure to ultraviolet (UV) rays, it is generally referred to as "photoaging." Further, naturally aged skin is smooth, pale, and finely wrinkled, while photoaged skin has thick skin wrinkles and causes pigmentation and telangiectasia.

Subcutaneous fat has an important role in maintaining energy homeostasis by secreting hormones and adipokines that control the metabolism of other tissues. Recently, it is known that the fat content of human subcutaneous adipose tissue is decreased by ultraviolet rays, which are environmental factors causing various diseases such as photoaging, inflammation, immune suppression, cancer, etc., and adiponectin as a fat-derived product is decreased in aged skin, while inducing an increase of MMP-1 and a decrease of collagen.

Sensitive skin refers to skin that reacts more sensitively than normal skin to external irritants, allergens, environmental changes, or internal causes of the human body, hence causing irritation reactions or dermatitis. As an inspection method for identifying the sensitive skin, there is an example of treating the skin with lactic acid or DMSO, which is known to cause dermatitis thus to determine the sensitive skin. Metabolic disease refers to a syndrome in which risk factors such as obesity, diabetes, hypertension, arteriosclerosis, and nonalcoholic fatty liver disease (NAFLD) appear together due to excessive nutrient accumulation in the body and lack of exercise, and recently, was officially named "metabolic syndrome" or "insulin resistance syndrome" through the Adult Treatment Program III established by the World Health Organization and the Heart, Lung, and Blood Institute of the US National Institutes of Health. Further, according to the ATP of the US National Cholesterol Education Program (NCEP) published in 2001, among five risk factors including: abdominal obesity with a waist size of 40 inches (102 cm) or more in men and 35 inches (88 cm) or more in women; triglycerides of 150 mg/dL or more; HDL cholesterol of 40 mg/dL or less in men and 50 mg/dL or less in women; blood pressure of 130/85 mmHg or more; and fasting glucose of 110 mg/dL or more, three or more factors appear, it is judged as a metabolic disease. In the case of Asians, the definition of abdominal obesity is somewhat adjusted as the waist size of 90 cm or more for men and 80 cm or more for women. If such regulations as described above are applied, there is a recent study report demonstrating that about 25% of total Korean population shows metabolic syndrome symptoms.

On the other hand, adiponectin is a type of adipokine, which is a protein hormone specifically secreted from adipocytes, and plays an important role in regulating cardiovascular diseases such as hyperglycemia, hyperinsulinemia, obesity, arteriosclerosis, etc. by enhancing the function of insulin and suppressing insulin resistance. Further, adiponectin has a function of inhibiting cancer cell metastasis and inflammatory response. Adiponectin not only proliferates keratinocytes but also promotes the expression of filaggrin, hyaluronic acid and extracellular matrix in the skin, thereby performing wound healing, fibrosis inhibition, skin wrinkle improvement, moisturizing, etc. Adiponectin is composed of 244 amino acids and consists of a signal sequence, a collagen-like domain located at N-terminus and a C1q-like globular domain located at C-terminus. A hexamer and a 400 kDa high molecular weight (HMW) complex are major oligomers, wherein the HMW complex is known to have higher activity than the low molecular weight (LMW) complex.

The development of adiponectin-derived peptides known to have different physiological activities has been the target of many domestic and foreign researchers and pharmaceutical companies. However, due to difficulties in formation of polymer in the body, final success possibility was relatively low.

Accordingly, there is a need for research on development of adiponectin-derived short peptides having excellent physiological activity, and studies on adiponectin-modified peptides that are advantageous for formulation into cosmetics and drugs.

### [Summary of Invention]

### [Problems to be Solved by Invention]

An object of the present invention is to provide a peptide having excellent solubility and stability.

Another object of the present invention is to provide a cosmetic composition including the peptide.

In addition, another object of the present invention is to provide a cosmetic composition for improving skin wrinkles, improving sensitive skin, improving dermatitis or promoting hair growth, which includes the peptide.

Further, another object of the present invention is to provide a pharmaceutical composition for improving skin wrinkles, improving dermatitis or promoting hair growth, which includes the peptide.

Furthermore, another object of the present invention is to provide a pharmaceutical composition for treatment of metabolic diseases, which includes the peptide.

### [Means for Solving Problems]

1. A peptide including an amino acid sequence SEQ ID NO: 1 or 2, and having an amino group at C-terminus.
2. The peptide according to the above 1, wherein at least one amino acid is a D-form.
3. The peptide according to the above 1, wherein one amino acid is a D-form.
4. A peptide including an amino acid sequence SEQ ID NO: 1 or 2, wherein at least one amino acid is a D-form.
5. The peptide according to the above 4, wherein one amino acid is a D-form.
6. A cosmetic composition including the peptide according to any one of the above 1 to 5.
7. The cosmetic composition according to the above 6, wherein the composition is adapted to improve skin wrinkles, improve sensitive skin, improve dermatitis or promote hair growth.
8. A pharmaceutical composition for improving skin wrinkles, improving dermatitis or promoting hair growth, including the peptide according to any one of the above 1 to 5.
9. A pharmaceutical composition for treatment of metabolic diseases, including the peptide according to any one of the above 1 to 5.

### [Advantageous effects]

The present invention relates to a peptide as well as a cosmetic composition and a pharmaceutical composition including the peptide, which have excellent solubility and stability such that they are advantageous for formulation when manufacturing cosmetics or drugs containing the same, thereby improving skin wrinkles, sensitive skin and dermatitis, promoting hair growth, and enhancing drug efficacy for metabolic diseases.

### [Brief Description of Drawings]

FIG. 1 illustrates cell permeability test results of a peptide of the present invention.
FIG.2 illustrates cytotoxicity test results of the peptide of the present invention.
FIG. 3 illustrates stability test results of the peptide of the present invention.
FIG. 4 illustrates *in vitro* AMPK-phosphorylation activity of the peptide of the present invention.
FIGS. 5 to 7 illustrate results of experiments on the efficacy of the peptide of the present invention for promoting adiponectin production, and procollagen and p-AMPK expression.
FIG. 8 illustrates confirmation of the ability of the peptide of the present invention for inhibiting accumulation of hepatocellular fat.
FIGS. 9 and 10 illustrate results of experiments on the efficacy of the peptide of the present invention for improving sensitive skin and dermatitis.
FIG. 11 illustrates results of experiments on the efficacy of the peptide of the present invention for promoting hair growth.

### [Mode for Carrying out Invention]

Hereinafter, the present invention will be described in detail.

The present invention provides a peptide including an amino acid sequence of SEQ ID NO: 1 or 2 and having an amino group at C-terminus.

The peptide of the present invention consists of an amino acid sequence of SEQ ID NO: 1 (LYYF) or SEQ ID NO: 2 (GLYYF) and has an amino group at C-terminus, thereby exhibiting enhanced solubility, cell permeability, toxicity and stability compared to the peptide having an -OH group at C-terminus.

The peptide may be one in which at least one amino acid is a D-form. At this time, regardless of the position of the amino acid, if at least one amino acid is a D-form, the above effect can be achieved.

The peptide of the present invention may be prepared by general chemical synthesis, for example, solid-phase peptide synthesis. Alternatively, the peptide of the present invention may be prepared by culturing a microorganism transformed with a recombinant vector containing the nucleic acid encoding the above peptide thus to express the peptide, followed by purification according to any conventional method, but it is not limited thereto.

The present invention may include functional equivalents and variants of a peptide which consists of the amino acid sequence of SEQ ID NO: 1 or 2 and has an amino group at C-terminus.

The functional equivalent of the peptide may include a peptide that does not change the activity of the peptide as a whole, specifically, a peptide having the same functional action may be included in the scope of the present invention. Further, the variant means, for example, a substance in which one or several amino acids is/are deleted, substituted or added in the amino acid sequence, and which has 95% or more, preferably 98% or more, and more preferably 99% or more identity to the amino acid sequence. Herein, the term "identity" refers to a ratio (%) of the number of identical amino acid residues in one amino acid sequence to the number of total amino acid residues, including the number of gaps, in the other amino acid sequence, when performing an alignment to reach the highest degree of identity with or without introducing the gaps into the above two amino acid sequences. Further, the term "several" means an integer of 2 to 10, for example, an integer of 2 to 7, 2 to 5, 2 to 4, or 2 to 3. Specific examples of the natural variant may include variants based on polymorphisms such as a single nucleotide polymorphism (SNP), splice variants and the like. Preferably, the substitution is a conservative amino acid substitution. This is because, if it is a conservative amino acid substitution, it may have a structure or property substantially equivalent to that of the peptide having the above amino acid sequence. Conservative amino acids known in the art may include, for example: mutually non-polar amino acids (glycine, alanine, phenylalanine, valine, leucine, isoleucine, methionine, proline, tryptophan); polar amino acids (amino acids other than non-polar amino acids); charged amino acids (acidic amino acids (aspartic acid, glutamic acid); basic amino acids (arginine, histidine, lysine); uncharged amino acids (amino acids other than charged amino acids); aromatic amino acids (phenylalanine, tryptophan, tyrosine); branched amino acids (leucine, isoleucine, valine); aliphatic amino acids (glycine, alanine, leucine, isoleucine, valine) and the like. Further, it may also include a peptide in which structural stability to heat, pH, etc. of the peptide is increased or peptide activity is increased by mutation or modification in the amino acid sequence.

The present invention provides a peptide having the amino acid sequence of SEQ ID NO: 1 or 2, wherein at least one amino acid is a D-form.

Since the peptide of the present invention consists of the amino acid sequence of SEQ ID NO: 1 (LYYF) or SEQ ID NO: 2 (GLYYF), in which at least one amino acid is a D-form, solubility, cell permeability, cytotoxicity and stability are improved as compared to peptides in which all amino acids are an L-form.

The peptide may be one in which one amino acid is a D-form. At this time, regardless of the position of the amino acid, if one amino acid is the D-form, the above effect can be achieved.

The peptide of the present invention can be prepared by general chemical synthesis, for example, solid-phase peptide synthesis. Alternatively, the peptide of the present invention may be prepared by culturing a microorganism transformed with a recombinant vector containing the nucleic acid encoding the above peptide thus to express the peptide, followed by purification according to any conventional method, but it is not limited thereto.

The present invention may include functional equivalents and variants of a peptide consisting of the amino acid sequence of SEQ ID NO: 1 or 2, wherein at least one amino acid is a D-form.

The functional equivalent of the peptide may include a peptide that does not change the activity of the peptide as a whole, specifically, a peptide having the same functional action may be included in the scope of the present invention. Further, the variant means, for example, a substance in which one or several amino acids is/are deleted, substituted or added in the amino acid sequence, and which has 95% or more, preferably 98% or more, and more preferably 99% or more identity to the amino acid sequence. Herein, the term "identity" refers to a ratio (%) of the number of identical amino acid residues in one amino acid sequence to the number of total amino acid residues, including the number of gaps, in the other amino acid sequence, when performing an alignment to reach the highest degree of identity with or without introducing the gaps into the above two amino acid sequences. Further, the term "several" means an integer of 2 to 10, for example, an integer of 2 to 7, 2 to 5, 2 to 4, or 2 to 3. Specific examples of the natural variant may include variants based on polymorphisms such as a single nucleotide polymorphism (SNP), splice variants and the like. Preferably, the substitution is a conservative amino acid substitution. This is because, if it is a conservative amino acid substitution, it may have a structure or property substantially equivalent to that of the peptide having the above amino acid sequence. Conservative amino acids known in the art may include, for example: mutually non-polar amino acids (glycine, alanine, phenylalanine, valine, leucine, isoleucine, methionine, proline, tryptophan); polar amino acids (amino acids other than non-polar amino acids); charged amino acids (acidic amino acids (aspartic acid, glutamic acid); basic amino acids (arginine, histidine, lysine); uncharged amino acids (amino acids other than charged amino acids); aromatic amino acids (phenylalanine, tryptophan, tyrosine); branched amino acids (leucine, isoleucine, valine); aliphatic amino acids (glycine, alanine, leucine, isoleucine, valine) and the like. Further, it may also include a peptide in which structural stability to heat, pH, etc. of the peptide is increased or peptide activity is increased by mutation or modification in the amino acid sequence.

The present invention provides a cosmetic composition including the peptide.

The cosmetic composition of the present invention may be adapted to improve skin wrinkles, improve sensitive skin, improve dermatitis or promote hair growth.

The sensitive skin may refer to skin appearing various hypersensitivity reactions caused by deterioration or abnormality in skin physiological function due to external or internal factors. Specifically, it refers to the skin that exhibits symptoms such as dry skin, rough skin, stinging, itching, allergic reactions, redness and erythema due to skin irritants that do not respond to non-sensitive skin, lack of sleep, overwork, changing seasons or stress.

The dermatitis may include a variety of inflammatory diseases appearing on the skin, for example, psoriatic dermatitis, dry dermatitis, atopic dermatitis, seborrheic dermatitis, contact dermatitis and molar dermatitis, but it is not limited thereto.

In the present invention, improvement refers to any process in which the symptoms are improved to some extent by the cosmetic composition of the present invention, and may mean prevention, alleviation and the like.

The hair growth promotion may include other terms used in the art, such as promotion of wool or hair growth, and may mean preventing or alleviating hair loss.

The cosmetic composition including the peptide of the present invention may further include components commonly used in the cosmetic composition, and may include, for example, conventional supplements such as antioxidants, stabilizers, solubilizing agent, vitamin, pigments and aromatic essences, as well as carriers.

Products to which the above composition can be added may include, for example, cosmetics such as astringent lotion, softening lotion, nourishing lotion, various creams, essence, pack, foundation, etc.; and cleansings, facial cleanser, soap, treatment, cosmetic liquids, etc., but they are not limited thereto.

Specific formulations of the cosmetic composition according to the present invention may include typical formulations such as skin lotion, skin softener, skin toner, astringent, lotion, milk lotion, moisture lotion, nourishing lotion, massage cream, nourishing cream, moisture cream, hand cream, essence, nourishing essence, pack, soap, shampoo, cleansing foam, cleansing lotion, cleansing cream, body lotion, body cleanser, emulsion, lipstick, makeup base, foundation, press powder, loose powder, eye shadow, rinse, gel, wax, mist, etc., but they are not limited thereto.

The composition may be formulated by incorporating the peptide inside the nanoliposome and stabilizing it. When the peptide is contained inside the nanoliposome, components of the peptide are stabilized to solve problems such as precipitation and deformation during formulation, and the solubility and percutaneous absorption of the components may be increased, thereby maximally expressing the efficacy expected from the peptide.

The present invention provides a pharmaceutical composition including the peptide.

The pharmaceutical composition of the present invention may be adapted to improve skin wrinkles, improve dermatitis, promote hair growth, or treat metabolic diseases.

The dermatitis may include, for example, psoriatic dermatitis, dry dermatitis, atopic dermatitis, seborrheic dermatitis, contact dermatitis and molar dermatitis, but it is not limited thereto.

In the present invention, improvement refers to any process in which the symptoms are improved to some extent due to administration of the pharmaceutical composition of the present invention, and may mean prevention, alleviation, treatment and the like.

The hair growth promotion may include other terms used in the art to promote wool or hair growth, and may mean preventing or treating hair loss.

Metabolic disease may be, for example, obesity, diabetes, complications of diabetes, fatty liver, dyslipidemia, insulin resistance, cardiovascular disease, arteriosclerosis, hyperglycemia, hyperlipidemia or abnormal carbohydrate metabolism, but it is not limited thereto.

The pharmaceutical composition including the peptide of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be added to formulate the pharmaceutical composition into injectable formulations, pills, capsules, granules or tablets such as aqueous solutions, suspensions, emulsions and the like.

The pharmaceutical composition of the present invention is applicable in a form of any formulation containing the peptide of the present invention as an active ingredient, and may be prepared in oral or parenteral formulations. The pharmaceutical formulations of the present invention may include forms suitable for oral, rectal, nasal, topical (including the cheek and sublingual), subcutaneous, vaginal or parenteral (including intramuscular, subcutaneous and intravenous) administration. Alternatively, forms suitable for administration by inhalation, insufflations or application may also be included, but it is not limited therebo.

The pharmaceutical composition of the present invention is administered in a pharmaceutically effective amount. Effective dose levels may be determined depending on types of disease of a patient, severity, activity of drug, sensitivity to drug, administration time, administration route and rate of release, duration of treatment, factors including concurrent medications, and other factors well known in the medical field.

The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The dosage of the pharmaceutical composition according to the present invention may vary widely depending on the weight, age, sex, health conditions or diet of the patient, administration time, administration method, excretion rate and severity of the disease, and the appropriate dosage depends on, for example, an amount of drug accumulated in the patient's body and/or specific efficacy of the peptide of the present invention used. Generally, the amount may be calculated on the basis of EC50, which is generally determined to be effective in i*n vivo* animal models and *in vitro,* for example, from 0.01 µg to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

The pharmaceutical composition of the present invention may further include a compound to maintain/increase one or more of active ingredients exhibiting the same or similar functions in relation to treatment of the above diseases or the solubility and/or absorption of at least one active ingredient. Further, the composition may also optionally include chemotherapeutic agents, anti-inflammatory agents, antiviral agents and/or immunomodulators and the like.

Further, the pharmaceutical composition of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

Hereinafter, the present invention will be described in detail with reference to the following examples.

### Example - Preparation of peptides

The peptides used in the present invention were synthesized by a solid phase method using 9-fluorenylmethoxycarbonyl (Fmoc) as a protective group of Nα-amino acid (Fmoc solid phase peptide synthesis), and N-hydroxybenzotriazole-diisopropylcarbodiimide (HOBt-DIC) method was used to extend the peptides (Wang C. Chan, Perter D. white, "Fmoc solid phase peptide synthesis" Oxford). The synthesized peptides were purified using high-performance liquid chromatography (Prep-HPLC, column C18, 10 µm, 250 mm × 22 mm) and lyophilized to obtain a powder form. Further, using liquid chromatography-mass spectrometry (LC-MS), molecular weights of the compounds of Table 1 were determined. A more detailed synthesis process is described below.

**[TABLE 1]**

| Peptide | Molecular weight |
|---|---|
| LYYF-NH₂ | 603.71 |
| GLYYF-NH₂ | 660.76 |
| lYYF | 604.69 |
| LyYF | 604.69 |
| LYyF | 604.69 |
| LYYf | 604.69 |
| GLYYF | 661.74 |
| GLyYF | 661.74 |
| GLYyF | 661.74 |
| GLYYf | 661.74 |
| lYYF-NH₂ | 603.71 |
| LyYF-NH₂ | 603.71 |
| LYyF-NH₂ | 603.71 |
| LYYf-NH₂ | 603.71 |
| GlYYF-NH₂ | 660.76 |
| GLyYF-NH₂ | 660.76 |
| GLYyF-NH₂ | 660.76 |
| GLYYf-NH₂ | 660.76 |

### 1. Synthesis of reactants

### (1) Synthesis of Fmoc-Phe-Rink Amide AM Resin (Compound 1a)

In a 50 ml reaction vessel, 50 ml of dimethylformamide (N,N-Dimethylformamide, DMF) containing 20% of Rink Amide AM Resin (1 mmol, 1.43 g) and piperidine was put and reacted at room temperature for 10 minutes. The reacted solution was removed by filtration and washed sequentially with 50 ml of dichloromethane (DCM), methyl alcohol, dichloromethane, and dimethylformamide, respectively. Fmoc-Phe-OH (N-Fmoc-L-phenylalanine) (8 mmol, 3.1 g), HOBt (8 mmol, 1.081 g) and DIC (8 mmol, 1.24 ml) dissolved in 50 ml of dimethylformamide were added to the product from which Fmoc was removed in a solid phase form through vacuum drying, followed by a reaction at room temperature for 4 hours. The reacted solution was removed by filtration, and the synthesized resin was sequentially washed with 50 ml of dichloromethane, methyl alcohol, dichloromethane, and dimethylformamide, respectively. Through vacuum drying, Compound 1a in a solid phase form was quantitatively obtained.

### (2) Synthesis of Fmoc-Tyr(tBu)-Phe-Rink Amide AM Resin (Compound 1b)

In a 50 ml reaction vessel, 50 ml of dimethylformamide (N,N-Dimethylformamide, DMF) containing 20% of Compound 1a and piperidine was put and reacted at room temperature for 10 minutes. The reacted solution was removed by filtration and washed sequentially with 50 ml of dichloromethane (DCM), methyl alcohol, dichloromethane, and dimethylformamide, respectively. Fmoc-Tyr(tBu)-OH (N-Fmoc-O-*tert*-butyl-L-tyrosine) (8 mmol, 3.68 g), HOBt (8 mmol, 1.081 g) and DIC (8 mmol, 1.24 ml) dissolved in 50 ml of dimethylformamide were added to the product from which Fmoc was removed in a solid phase form through vacuum drying, followed by a reaction at room temperature for 4 hours. The reacted solution was removed by filtration, and the synthesized resin was sequentially washed with 50 ml of dichloromethane, methyl alcohol, dichloromethane, and dimethylformamide, respectively. Through vacuum drying, Compound 1b in a solid phase form was quantitatively obtained.

### (3) Synthesis of Fmoc-Tyr(tBu)-Tyr(tBu)-Phe-Rink Amide AM Resin (Compound 1c)

Except that 50 ml of dimethylformamide (N,N-Dimethylformamide, DMF) containing 20% of Compound 1b and piperidine was put in a 50 ml reaction vessel and reacted at room temperature for 10 minutes, the following processes are substantially the same as described in Example (2).

### (4) Synthesis of Fmoc-Leu-Tyr(tBu)-Tyr(tBu)-Phe-Rink Amide AM Resin (Compound 1d)

In a 50 ml reaction vessel, 50 ml of dimethylformamide (N,N-Dimethylformamide, DMF) containing 20% of Compound 1c and piperidine was added and reacted at room temperature for 10 minutes. The reacted solution was removed by filtration and washed sequentially with 50 ml of dichloromethane (DCM), methyl alcohol, dichloromethane, and dimethylformamide, respectively. Fmoc-Leu-OH (N-Fmoc-L-leucine) (8 mmol, 3.68 g), HOBt (8 mmol, 1.081 g) and DIC (8 mmol, 1.24 ml) dissolved in 50 ml of dimethylformamide were added to the product from which Fmoc was removed in a solid phase form through vacuum drying, followed by a reaction at room temperature for 4 hours. The reacted solution was removed by filtration, and the synthesized resin was sequentially washed with 50 ml of dichloromethane, methyl alcohol, dichloromethane, and dimethylformamide, respectively. Through vacuum drying, Compound 1d in a solid phase form was quantitatively obtained.

### (5) Synthesis of Fmoc-Gly-Leu-Tyr(tBu)-Tyr(tBu)-Phe-Rink Amide AM Resin (Compound 2a)

In a 50 ml reaction vessel, 50 ml of dimethylformamide (N,N-Dimethylformamide, DMF) containing 20% of Compound 1d and piperidine was put and reacted at room temperature for 10 minutes. The reacted solution was removed by filtration and washed sequentially using 50 ml of dichloromethane (DCM), methyl alcohol, dichloromethane, and dimethylformamide, respectively. Fmoc-Gly-OH (Fmoc-Glycine) (8 mmol, 2.38 g), HOBt (8 mmol, 1.081 g) and DIC (8 mmol, 1.24 ml) dissolved in 50 ml of dimethylformamide were added to the product from which Fmoc was removed in a solid phase form through vacuum drying, followed by a reaction at room temperature for 4 hours. The reacted solution was removed by filtration, and the synthesized resin was sequentially washed using 50 ml of dichloromethane, methyl alcohol, dichloromethane, and dimethylformamide, respectively. Through vacuum drying, Compound 2a in a solid phase form was quantitatively obtained.

### (6) Synthesis of Fmoc-Phe-2-Chloro-Trityl Resin (Compound 3a)

In a 50 ml reaction vessel, 2-chlorotrityl chloride resin (1 mmol, 0.714 g) and Fmoc-Phe-OH (N-Fmoc-L-phenylalanine) (2 mmol, 0.78 g), diisopropyl ethylamine (N,N -Diisopropylethylamine) (6 mmol, 1.05 ml) dissolved in 50 ml of dichloromethane were added, followed by a reaction at room temperature for 6 hours. The reacted solution was removed by filtration, and the synthesized resin was sequentially washed with 50 ml of dichloromethane (DCM), methyl alcohol, dichloromethane, and dimethylformamide, respectively. Through vacuum drying, Compound 3a in a solid phase form was quantitatively obtained.

### (7) Synthesis of Fmoc-Tyr (tBu)-Phe-2-Chloro-Trityl Resin (Compound 3b)

Except that 50 ml of dimethylformamide (N,N-Dimethylformamide, DMF) containing 20% of Compound 3a and piperidine was put in a 50 ml reaction vessel and reacted at room temperature for 10 minutes, the following processes are substantially the same as described in Example (2).

### (8) Synthesis of Fmoc-Tyr(tBu)-Tyr(tBu)-Phe-2-ChloroTrityl Resin (Compound 3c)

Except that 50 ml of dimethylformamide (N,N-Dimethylformamide, DMF) containing 20% of Compound 3b and piperidine was put in a 50 ml reaction vessel and reacted at room temperature for 10 minutes, the following processes are substantially the same as described in Example (2).

### (9) Synthesis of Fmoc-D-Leu-Tyr(tBu)-Tyr(tBu)-Phe-2-Chloro-Trityl Resin (Compound 3d)

In a 50 ml reaction vessel, 50 ml of dimethylformamide (N,N-Dimethylformamide, DMF) containing 20% of Compound 3c and piperidine was put and reacted at room temperature for 10 minutes. The reacted solution was removed by filtration, and was washed sequentially with 50 ml of dichloromethane (DCM), methyl alcohol, dichloromethane, and dimethylformamide, respectively. Fmoc-D-Leu-OH (N-Fmoc-D-leucine) (8 mmol, 3.68 g), HOBt (8 mmol, 1.081 g) and DIC (8 mmol, 1.24 ml) dissolved in 50 ml of dimethylformamide were added to the product from which Fmoc in a solid phase form was removed through vacuum drying, followed by a reaction at room temperature for 4 hours. The reacted solution was removed by filtration, and the synthesized resin was sequentially washed using 50 ml of dichloromethane, methyl alcohol, dichloromethane, and dimethylformamide, respectively. Through vacuum drying, Compound 3d in a solid phase form was quantitatively obtained.

### (10) Synthesis of Fmoc-D-Tyr(tBu)-Tyr(tBu)-Phe-2-Chloro-Trityl Resin (Compound 4a)

In a 50 ml reaction vessel, 50 ml of dimethylformamide (N,N-Dimethylformamide, DMF) containing 20% of Compound 3b and piperidine was put and reacted at room temperature for 10 minutes. The reacted solution was removed by filtration and washed sequentially with 50 ml of dichloromethane (DCM), methyl alcohol, dichloromethane, and dimethylformamide, respectively. Fmoc-D-Tyr(tBu)-OH (N-Fmoc-O-*tert*-butyl-D-tyrosine) (8 mmol, 3.68 g), HOBt (8 mmol, 1.081 g) and DIC (8 mmol, 1.24 ml) dissolved in 50 ml of dimethylformamide was added to the product from which Fmoc in a solid phase form was removed through vacuum drying, followed by a reaction at room temperature for 4 hours. The reacted solution was removed by filtration, and the synthesized resin was sequentially washed with 50 ml of dichloromethane, methyl alcohol, dichloromethane, and dimethylformamide, respectively. Through vacuum drying, Compound 4a in a solid phase form was quantitatively obtained.

### (11) Synthesis of Fmoc-Leu-D-Tyr(tBu)-Tyr(tBu)-Phe-2-Chloro-Trityl Resin (Compound 4b)

Except that 50 ml of dimethylformamide (N,N-Dimethylformamide, DMF) containing 20% of Compound 4a and piperidine was put in a 50 ml reaction vessel and reacted at room temperature for 10 minutes, the following processes are substantially the same as described in Example (4).

### (12) Synthesis of Fmoc-D-Tyr (tBu)-Phe-2-ChloroTrityl Resin (Compound 5a)

Except that 50 ml of dimethylformamide (N,N-Dimethylformamide, DMF) containing 20% of Compound 3a and piperidine was put in a 50 ml reaction vessel and reacted at room temperature for 10 minutes, the following processes are substantially the same as described in Example (10).

### (13) Synthesis of Fmoc-Tyr(tBu)-D-Tyr(tBu)-Phe-2-Chloro-Trityl Resin (Compound 5b)

Except that 50 ml of dimethylformamide (N,N-Dimethylformamide, DMF) containing 20% of Compound 5a and piperidine was put in a 50 ml reaction vessel and reacted at room temperature for 10 minutes, the following processes are substantially the same as described in Example (2).

### (14) Synthesis of Fmoc-Leu-Tyr(tBu)-D-Tyr(tBu)-Phe-2-Chloro-Trityl Resin (Compound 5c)

Except that 50 ml of dimethylformamide (N,N-Dimethylformamide, DMF) containing 20% of Compound 5b and piperidine was put in a 50 ml reaction vessel and reacted at room temperature for 10 minutes, the following processes are substantially the same as described in Example (4).

### (15) Synthesis of Fmoc-D-Phe-2-Chloro-Trityl Resin (Compound 6a)

In a 50 ml reaction vessel, 2-chlorotrityl chloride resin (1 mmol, 0.714 g) and Fmoc-D-Phe-OH (N-Fmoc-D-phenylalanine) (2 mmol, 0.78 g) and diisopropyl ethylamine (N,N -Diisopropylethylamine) (6 mmol, 1.05 ml) dissolved in 50 ml of dichloromethane were added, followed by a reaction at room temperature for 6 hours. The reacted solution was removed by filtration, and the synthesized resin was sequentially washed with 50 ml of dichloromethane (DCM), methyl alcohol, dichloromethane, and dimethylformamide, respectively. Through vacuum drying, Compound 6a in a solid phase form was quantitatively obtained.

### (16) Synthesis of Fmoc-Tyr(tBu)-D-Phe-2-Chloro-Trityl Resin (Compound 6b)

Except that 50 ml of dimethylformamide (N,N-Dimethylformamide, DMF) containing 20% of Compound 6a and piperidine was put in a 50 ml reaction vessel and reacted at room temperature for 10 minutes, the following processes are substantially the same as described in Example (2).

### (17) Synthesis of Fmoc-Tyr(tBu)-Tyr(tBu)-D-Phe-2-Chloro-Trityl Resin (Compound 6c)

Except that 50 ml of dimethylformamide (N,N-Dimethylformamide, DMF) containing 20% of Compound 6b and piperidine was put in a 50 ml reaction vessel and reacted at room temperature for 10 minutes, the following processes are substantially the same as described in Example (2).

### (18) Synthesis of Fmoc-Leu-Tyr(tBu)-Tyr(tBu)-D-Phe-2-Chloro-Trityl Resin (Compound 6d)

Except that 50 ml of dimethylformamide (N,N-Dimethylformamide, DMF) containing 20% of Compound 6c and piperidine was put in a 50 ml reaction vessel and reacted at room temperature for 10 minutes, the following processes are substantially the same as described in Example (4).

### (19) Synthesis of Fmoc-Gly-D-Leu-Tyr(tBu)-Tyr(tBu)-Phe-2-Chloro-Trityl Resin (Compound 7a)

Except that 50 ml of dimethylformamide (N,N-Dimethylformamide, DMF) containing 20% of Compound 3d and piperidine was put in a 50 ml reaction vessel and reacted at room temperature for 10 minutes, the following processes are substantially the same as described in Example (5).

### (20) Synthesis of Fmoc-Gly-Leu-D-Tyr(tBu)-Tyr(tBu)-Phe-2-Chloro-Trityl Resin (Compound 8a)

Except that 50 ml of dimethylformamide (N,N-Dimethylformamide, DMF) containing 20% of Compound 4b and piperidine was put in a 50 ml reaction vessel and reacted at room temperature for 10 minutes, the following processes are substantially the same as described in Example (5).

### (21) Synthesis of Fmoc-Gly-Leu-Tyr(tBu)-D-Tyr(tBu)-Phe-2-Chloro-Trityl Resin (Compound 9a)

Except that 50 ml of dimethylformamide (N,N-Dimethylformamide, DMF) containing 20% of Compound 5c and piperidine was put in a 50 ml reaction vessel and reacted at room temperature for 10 minutes, the following processes are substantially the same as described in Example (5).

### (22) Synthesis of Fmoc-Gly-Leu-Tyr(tBu)-Tyr(tBu)-D-Phe-2-Chloro-Trityl Resin (Compound 10a)

Except that 50 ml of dimethylformamide (N,N-Dimethylformamide, DMF) containing 20% of Compound 6d and piperidine was put in a 50 ml reaction vessel and reacted at room temperature for 10 minutes, the following processes are substantially the same as described in Example (5).

### (23) Synthesis of Fmoc-D-Leu-Tyr(tBu)-Tyr(tBu)-Phe-Rink Amide AM Resin (Compound 11a)

Synthesis of Fmoc-D-Leu-Tyr(tBu)-Tyr(tBu)-Phe-Rink Amide AM Resin (11a): In a 50 ml reaction vessel, 50 ml of dimethylformamide containing 20% of Compound 1c and piperidine was put and reacted for 10 minutes at room temperature. The reacted solution was removed by filtration and washed sequentially with 50 ml of dichloromethane, methyl alcohol, dichloromethane, and dimethylformamide, respectively. Fmoc-D-Leu-OH (N-Fmoc-D-leucine) (8 mmol, 3.68 g), HOBt (8 mmol, 1.081 g) and DIC (8 mmol, 1.24 ml) dissolved in 50 ml of dimethylformamide were added to the product from which Fmoc in a solid phase form was removed through vacuum drying, followed by a reaction at room temperature for 4 hours. The reacted solution was removed by filtration, and the synthesized resin was sequentially washed with 50 ml of dichloromethane, methyl alcohol, dichloromethane, and dimethylformamide, respectively. Through vacuum drying, Compound 11a (Fmoc-D-Leu-Tyr(tBu)-Tyr(tBu)-Phe-Rink Amide AM Resin) in a solid phase form was quantitatively obtained.

### (24) Synthesis of Fmoc-D-Tyr(tBu)-Tyr(tBu)-Phe-Rink Amide AM Resin (Compound 12a)

Except that 50 ml of dimethylformamide (N,N-Dimethylformamide, DMF) containing 20% of Compound 1b and piperidine was put in a 50 ml reaction vessel and reacted at room temperature for 10 minutes, the following processes are substantially the same as described in Example (10).

### (25) Synthesis of Fmoc-Leu-D-Tyr(tBu)-Tyr(tBu)-Phe-Rink Amide AM Resin (Compound 12b)

Except that 50 ml of dimethylformamide (N,N-Dimethylformamide, DMF) containing 20% of Compound 12a and piperidine was put in a 50 ml reaction vessel and reacted at room temperature for 10 minutes, the following processes are substantially the same as described in Example (4).

### (26) Synthesis of Fmoc-D-Tyr(tBu)-Phe-Rink Amide AM Resin (Compound 13a)

Except that 50 ml of dimethylformamide (N,N-Dimethylformamide, DMF) containing 20% of Compound 1a and piperidine was put in a 50 ml reaction vessel and reacted at room temperature for 10 minutes, the following processes are substantially the same as described in Example (10).

### (27) Synthesis of Fmoc-Tyr(tBu)-D-Tyr(tBu)-Phe-Rink Amide AM Resin (Compound 13b)

Except that 50 ml of dimethylformamide (N,N-Dimethylformamide, DMF) containing 20% of Compound 13a and piperidine was put in a 50 ml reaction vessel and reacted at room temperature for 10 minutes, the following processes are substantially the same as described in Example (2).

### (28) Synthesis of Fmoc-Leu-Tyr(tBu)-D-Tyr(tBu)-Phe-Rink Amide AM Resin (Compound 13c)

Except that 50 ml of dimethylformamide (N,N-Dimethylformamide, DMF) containing 20% of Compound 13b and piperidine was put in a 50 ml reaction vessel and reacted at room temperature for 10 minutes, the following processes are substantially the same as described in Example (4).

### (29) Synthesis of Fmoc-D-Phe-Rink Amide AM Resin (Compound 14a)

In a 50 ml reaction vessel, 50 ml of dimethylformamide (N,N-Dimethylformamide, DMF) containing 20% of Rink Amide AM Resin (1 mmol, 1.43 g) and piperidine was put and reacted for 10 minutes at room temperature. The reacted solution was removed by filtration and washed sequentially with 50 ml of dichloromethane (DCM), methyl alcohol, dichloromethane, and dimethylformamide, respectively. Fmoc-D-Phe-OH (N-Fmoc-D-phenylalanine) (8 mmol, 3.1 g), HOBt (8 mmol, 1.081 g) and DIC (8 mmol, 1.24 ml) dissolved in 50 ml of dimethylformamide were added to the product from which Fmoc in a solid phase form was removed through vacuum drying, followed by a reaction at room temperature for 4 hours. The reacted solution was removed by filtration, and the synthesized resin was sequentially washed with 50 ml of dichloromethane, methyl alcohol, dichloromethane, and dimethylformamide, respectively. Through vacuum drying, Compound 14a in a solid phase form was quantitatively obtained.

### (30) Synthesis of Fmoc-Tyr(tBu)-D-Phe-Rink Amide AM Resin (Compound 14b)

Except that 50 ml of dimethylformamide (N,N-Dimethylformamide, DMF) containing 20% of Compound 14a and piperidine was put in a 50 ml reaction vessel and reacted at room temperature for 10 minutes, the following processes are substantially the same as described in Example (2).

### (31) Synthesis of Fmoc-Tyr(tBu)-Tyr(tBu)-D-Phe-Rink Amide AM Resin (Compound 14c)

Except that 50 ml of dimethylformamide (N,N-Dimethylformamide, DMF) containing 20% of Compound 14b and piperidine was put in a 50 ml reaction vessel and reacted at room temperature for 10 minutes, the following processes are substantially the same as described in Example (2).

### (32) Synthesis of Fmoc-Leu-Tyr(tBu)-Tyr(tBu)-D-Phe-Rink Amide AM Resin (Compound 14d)

Except that 50 ml of dimethylformamide (N,N-Dimethylformamide, DMF) containing 20% of Compound 14c and piperidine was put in a 50 ml reaction vessel and reacted at room temperature for 10 minutes, the following processes are substantially the same as described in Example (4).

### (33) Synthesis of Fmoc-Gly-D-Leu-Tyr(tBu)-Tyr(tBu)-Phe-Rink Amide AM Resin (Compound 15a)

Except that 50 ml of dimethylformamide (N,N-Dimethylformamide, DMF) containing 20% of Compound 11a and piperidine was put in a 50 ml reaction vessel and reacted at room temperature for 10 minutes, the following processes are substantially the same as described in Example (5).

### (34) Synthesis of Fmoc-Gly-Leu-D-Tyr(tBu)-Tyr(tBu)-Phe-Rink Amide AM Resin (Compound 16a)

Except that 50 ml of dimethylformamide (N,N-Dimethylformamide, DMF) containing 20% of Compound 12b and piperidine was put in a 50 ml reaction vessel and reacted at room temperature for 10 minutes, the following processes are substantially the same as described in Example (5).

### (35) Synthesis of Fmoc-Gly-Leu-Tyr(tBu)-D-Tyr(tBu)-Phe-Rink Amide AM Resin (Compound 17a)

Except that 50 ml of dimethylformamide (N,N-Dimethylformamide, DMF) containing 20% of Compound 13c and piperidine was put in a 50 ml reaction vessel and reacted at room temperature for 10 minutes, the following processes are substantially the same as described in Example (5).

### (36) Synthesis of Fmoc-Gly-Leu-Tyr(tBu)-Tyr(tBu)-D-Phe-Rink Amide AM Resin (Compound 18a)

Except that 50 ml of dimethylformamide (N,N-Dimethylformamide, DMF) containing 20% of Compound 14d and piperidine was put in a 50 ml reaction vessel and reacted at room temperature for 10 minutes, the following processes are substantially the same as described in Example (5).

### 2. Synthesis of peptides

50 ml of dimethylformamide containing 20% of piperidine as well as any one of the compounds synthesized in Example 1 (Table 2) was put in a 50 ml reaction vessel and reacted at room temperature for 10 minutes. The reacted solution was removed by filtration, and washed with 50 ml of dichloromethane three times. To the product from which Fmoc in a solid phase form was removed through vacuum drying, 50 ml of a mixture for separating a protective group (trifluoroacetic acid: triisopropylsilane: water = 95: 2.5: 2.5) as well as resin was added, followed by a reaction at room temperature for 3 hours. The reacted solution was collected by filtration, and 500 ml of diethyl ether was added thereto to precipitate the product. The solid product was collected using a centrifuge, washed twice with 500 ml of diethyl ether, and then dried. The obtained solid product was purified using Prep-HPLC (column C18, 10 um, 250 mm × 22 mm), and then lyophilized to obtain each peptide.

**[TABLE 2]**

| Peptide | Reactant |
|---|---|
| LYYF-NH₂ | Compound 1d |
| GLYYF-NH₂ | Compound 2a |
| lYYF | Compound 3d |
| LyYF | Compound 4b |
| LYyF | Compound 5c |
| LYYf | Compound 6d |
| GlYYF | Compound 7a |
| GLyYF | Compound 8a |
| GLyYF | Compound 9a |
| GLYYf | Compound 10a |
| lYYF-NH₂ | Compound 11a |
| LyYF-NH₂ | Compound 12b |
| LYyF-NH₂ | Compound 13c |
| LYYf-NH₂ | Compound 14d |
| GlYYF-NH₂ | Compound 15a |
| GLyYF-NH₂ | Compound 16a |
| GLYyF-NH₂ | Compound 17a |
| GLYYf-NH₂ | Compound 18a |

### Experimental Example

### 1. Determination of solubility

To 5 mg of each of the peptides synthesized by the above method, a small amount of water or butylene glycol was added to measure solubility.

Results thereof are shown in Table 3 below.

**[TABLE 3]**

| No. | | Peptide modification | Solubility | Solvent | Mw | Note |
|---|---|---|---|---|---|---|
| CNT | | Control | | H2O (watersoluble) | | |
| Veh | | Vehicle | | BG (lipo-soluble) | | |
| P4-1 | LYYF-NH₂ | NH₂ | 5 mg/ml (5000 ppm) | H2O | 604.21 | Single solvent |
| P4-2 | lYYF | D-form (lower case: l.c.) | 5 mg/ml (5000 ppm) | H2O | 605.17 | Single solvent |
| P4-3 | LyYF | D-form (lower case: l.c.) | 5 mg/ml (5000 ppm) | H2O | 605.23 | Single solvent |
| P4-4 | LYyF | D-form (lower case: l.c.) | 5 mg/ml (5000 ppm) | H2O | 605.3 | Single solvent |
| P4-5 | LYYf | D-form (lower case: l.c.) | 2.5 mg/ml | H2O | 605.21 | Single solvent |
| P4-6 | lYYF-NH₂ | D-form (lower case: l.c.), NH₂ | 4 mg/ml | H2O:BG | 604.27 | Mixed solvent |
| P4-7 | LyYF-NH₂ | D-form (lower case: l.c.), NH₂ | 5 mg/ml (5000 ppm) | BG | 604.34 | Single solvent |
| P4-8 | LYyF-NH₂ | D-form (lower case: l.c.), NH₂ | 5 mg/ml (5000 ppm) | BG | 604.41 | Single solvent |
| P4-9 | LYYf-NH₂ | D-form (lower case: l.c.), NH₂ | 4 mg/ml | H2O:BG | 604.41 | Mixed solvent |
| P5-1 | GlYYF | D-form (lower case: l.c.) | 3 mg/ml | BG | 662.3 | Single solvent |
| P5-2 | GLyYF | D-form (lower case: l.c.) | 5 mg/ml (5000 ppm) | BG | 662.3 | Single solvent |
| P5-3 | GLYyF | D-form (lower case: l.c.) | 3 mg/ml | H2O:BG | 662.36 | Mixed solvent |
| P5-4 | GLYYF | D-form (lower case: l.c.) | 3 mg/ml | BG | 662.24 | Single solvent |
| P5-5 | GLYYF-NH₂ | NH₂ | 5 mg/ml (5000 ppm) | H2O | 661.4 | Single solvent |
| P5-6 | GlYYF-NH₂ | D-form (lower case: l.c.), NH₂ | 5 mg/ml (5000 ppm) | BG | 661.31 | Single solvent |
| P5-7 | GLyYF-NH₂ | D-form (lower case: l.c.), NH₂ | 5 mg/ml (5000 ppm) | BG | 661.32 | Single solvent |
| P5-8 | GLYyF-NH₂ | D-form (lower case: l.c.), NH₂ | 3 mg/ml | BG | 661.32 | Single solvent |
| P5-9 | GLYYf-NH₂ | D-form (lower case: l.c.), NH₂ | 5 mg/ml (5000 ppm) | BG | 661.47 | Single solvent |
| P5-5Bu | GLYYF-NH₂ | NH₂ | 5 mg/ml (5000 ppm) | BG | 661.4 | Single solvent |
| P5 | GLYYF | Original | 1 mg/ml | EtOH:PG | 662 | Mixed solvent |

| | | | | | | |
|---|---|---|---|---|---|---|
| * Herein, BG is butylene glycol and PG is propylene glycol | | | | | | |

### 2. Measurement of cell permeability

The skin permeability of each peptide synthesized by the above method was verified using pig skin, which most similar to human skin and thus is generally used for testing permeability. After cutting the pig skin into a size of 2 cm in width and height, FITC fluorescence-attached peptide stock (positive control P5: GLYYF peptide) and DMSO were prepared by concentration. Then, the prepared solution was applied in a sufficient amount to the pig skin. The applied pig skin was placed in an incubator at 37 °C for 2 hours while light shielding. After rapid freezing using LN2, cryosection was performed. After treatment with DAPI (nuclear staining) for 5 minutes, it was washed and then observed with a confocal microscope.

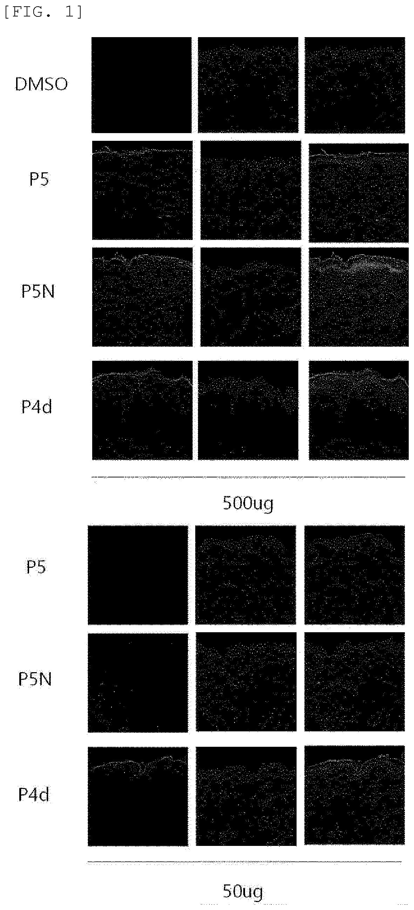

As a result, it was confirmed that the peptides of the present invention (P5N: GLYYF-NH₂, P4d: LYyF) had superior permeability to the pig skin compared to the P5: GLYYF peptide used as a control (FIG. 1).

### 3. Confirmation of cytotoxicity

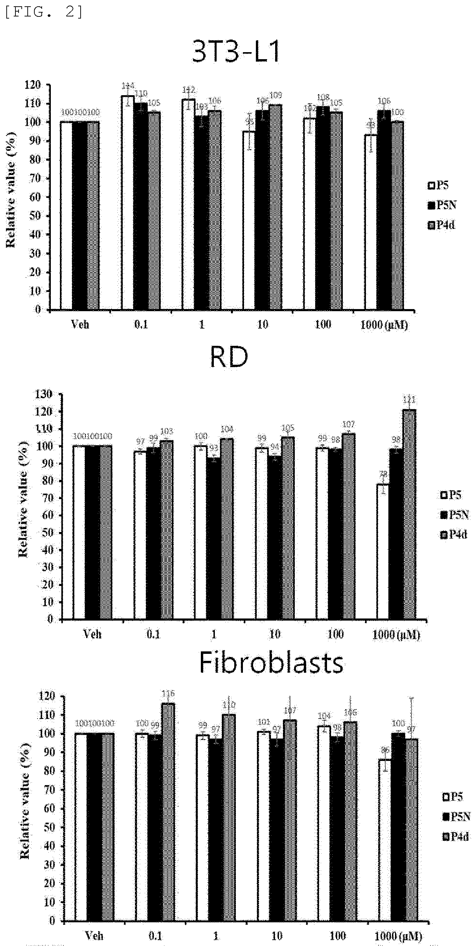

Using 3 types of cell lines (3T3-L1 adipocytes, RD muscle cells, and fibroblasts), each cell was seeded in a 24-well plate by 5 × 10⁴ cells per well and cultured for 24 hours in each cell culture condition. The medium was discarded and the cultured product was washed with PBS, followed by replacing with a new medium not containing 10% FBS. The product was treated with each peptide by concentration and incubated for 24 hours. After carefully removing the medium and washing the product with PBS, MTT reagent was added according to the manufacturer's method. Then, after reacting at room temperature for 30 minutes, absorbance was measured at 450 nm to confirm that no toxicity by each peptide was found in all cell lines (FIG. 2) .

### 4. Confirmation of stability

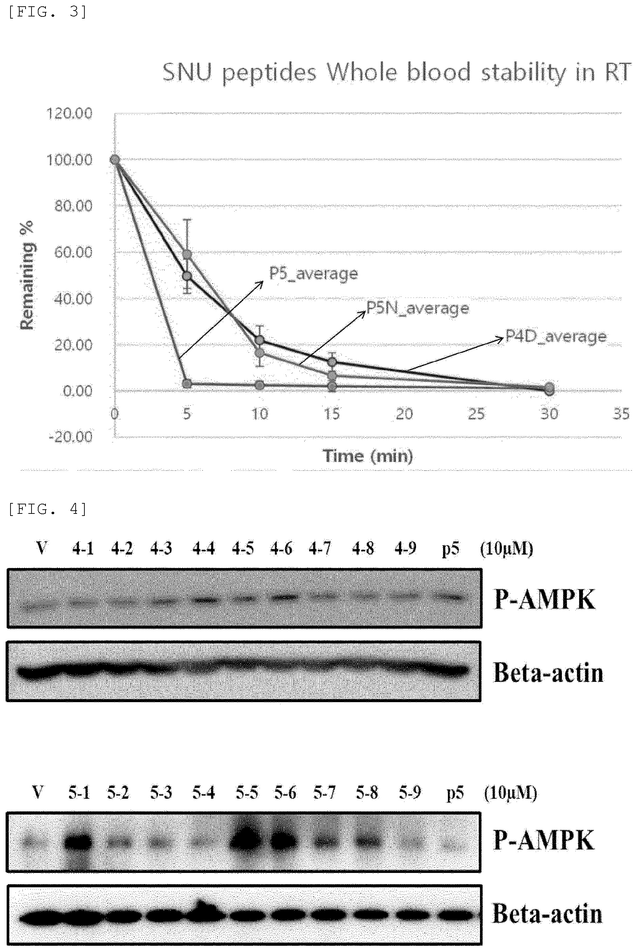

Mouse whole blood was treated with peptide (1 mg/ml) and reacted for 0, 15, 30, 60, and 120 minutes, followed by analyzing the peptides remaining as LC-QTOF_MS. As a result, it was confirmed that the peptide of the present invention (P5N: GLYYF-NH₂, P4d: LYyF) has improved stability compared to the P5: GLYYF peptide used as a control (FIG. 3).

### 5. Confirmation of in vitro AMPK-phosporylation activity

The mouse adipocyte line 3T3-L1 was treated with each peptide at a concentration of 10 µM and cultured for 24 hours thus to obtain the cells. Then, the protein was extracted and the effect on AMPK phosphorylation, which is responsible for the main sub-signaling of adiponectin, was compared and confirmed by Western blot (FIG. 4). As a result, it was confirmed that the peptides of the present invention increase phosphorylation of AMPK (V: DMSO, p5: GLYYF) .

### 6. Confirmation of efficacy in promoting adiponectin generation, matrix protein collagen and p-AMPK expression when applied to endogenous aged skin

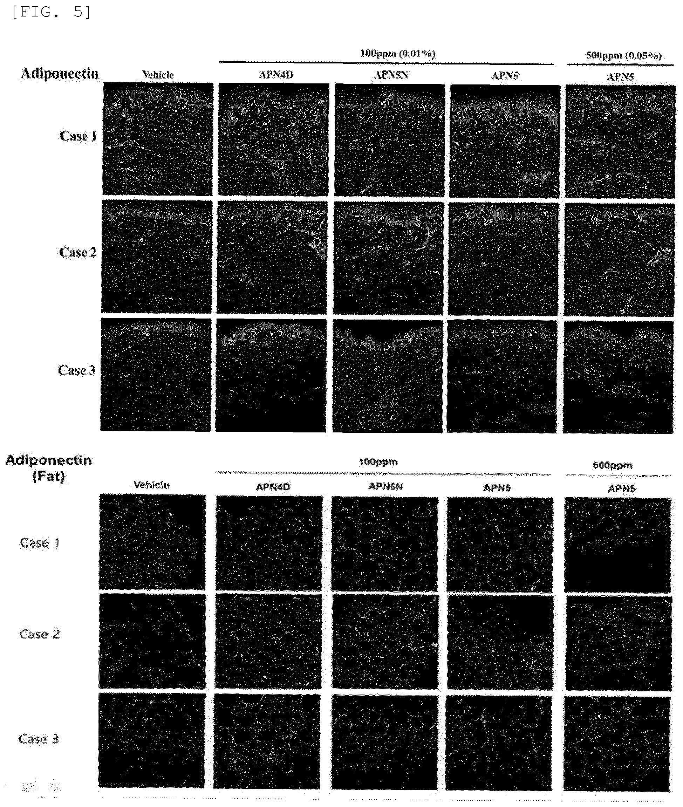

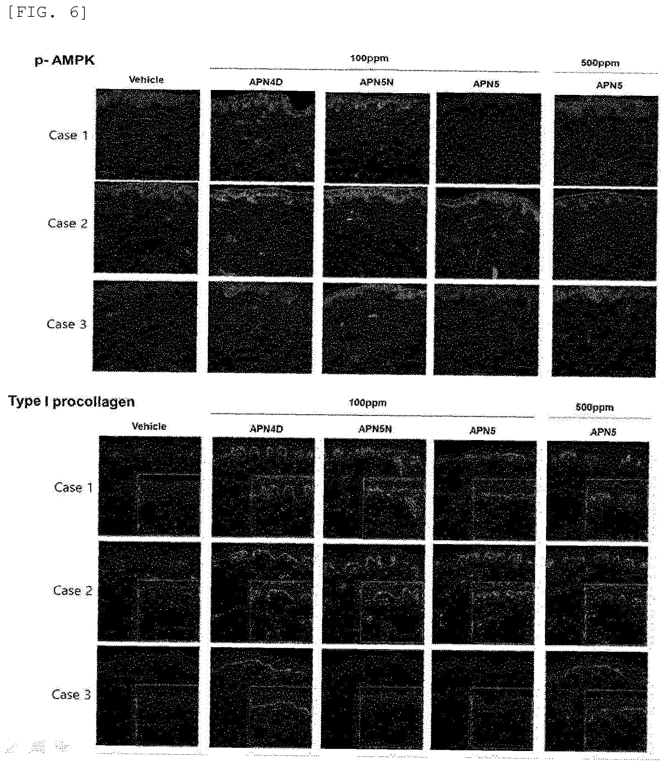

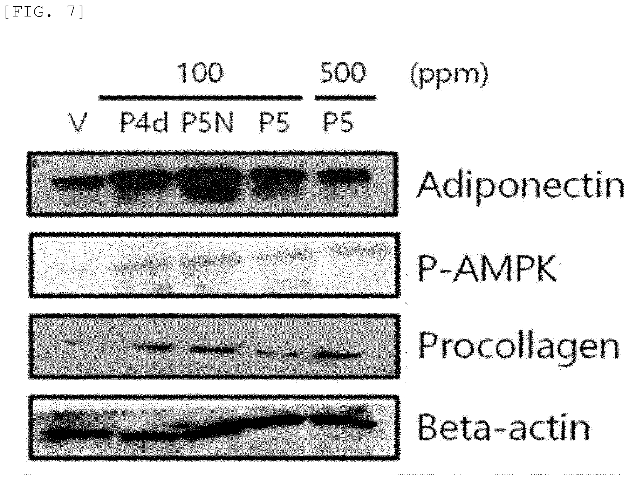

After applying the peptide of the present invention to the elderly skin reported to have reduced adiponectin, a biopsy was performed 24 hours later. As a result of observing the expression of adiponectin, matrix protein procollagen and p-AMPK through Western blot and immunochemical staining, the expression of adiponectin, procollagen and p-AMPK was increased in the peptide of the present invention compared to the negative control (vehicle) and the existing P5 peptide (FIGS. 5 to 7).

### 7. Confirmation of hepatocellular fat accumulation inhibitory ability

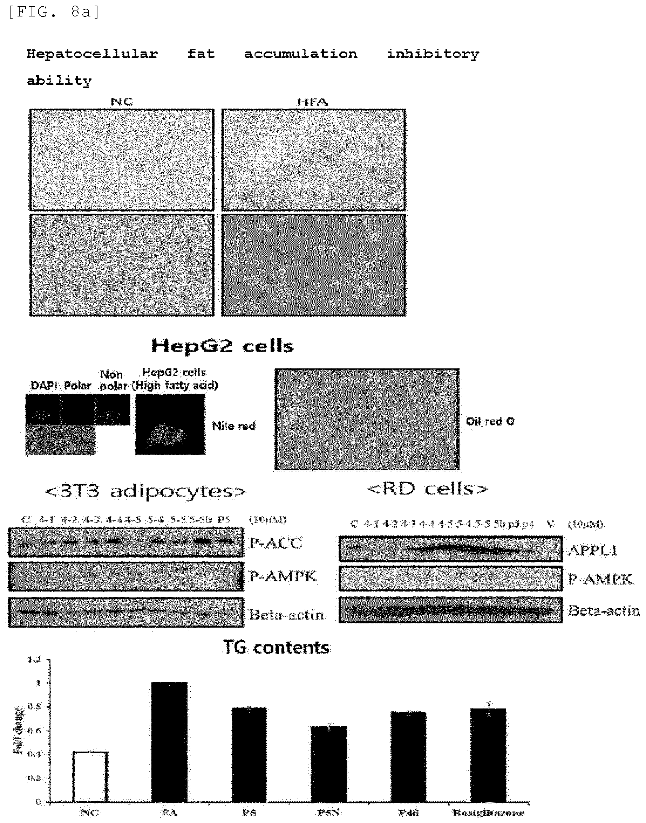

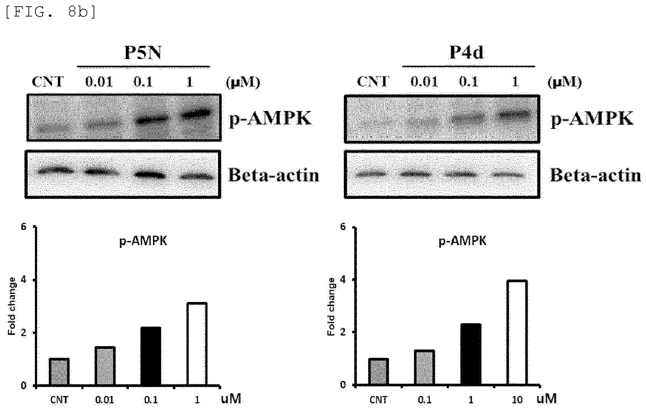

The liver cancer cell line, that is, HepG2 cells were treated with 1 mM free fatty acid (FFA) and 10 µM of peptide. As a result of triglyceride content comparison, a fat accumulation inhibitory ability of the peptide of the present invention was superior to that of rosiglitazone and P5, which are positive controls (FIG. 8). Further, with regard to acetyl-CoA carboxylase (ACC), which is a major enzyme for synthesizing triglycerides, and AMPK, which is a sub-signal regulator of adiponectin responsible for its regulation, phosphorylation was increased (p-ACC is inactive and inhibits triglyceride synthesis).

### 8. Confirmation of sensitive skin, dermatitis improvement efficacy

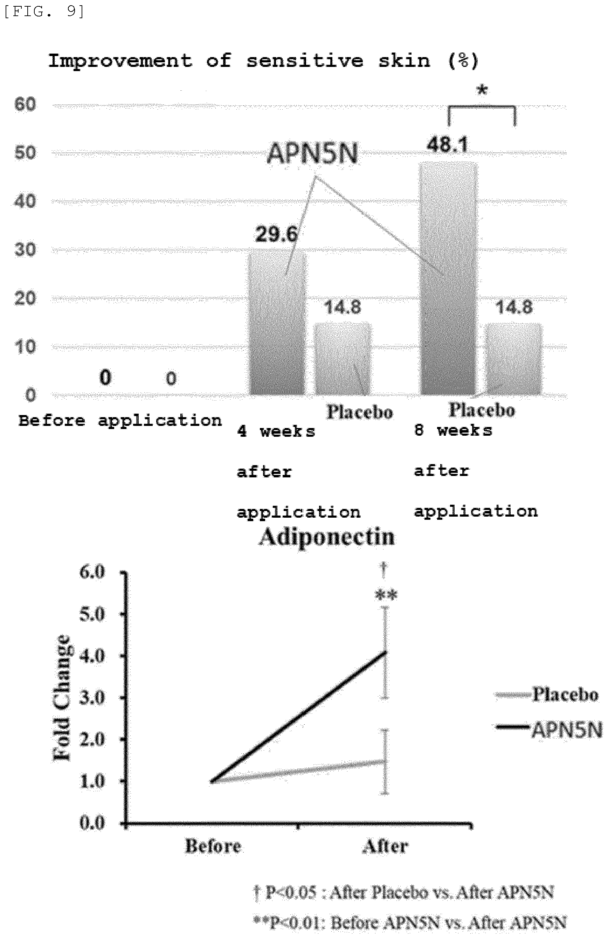

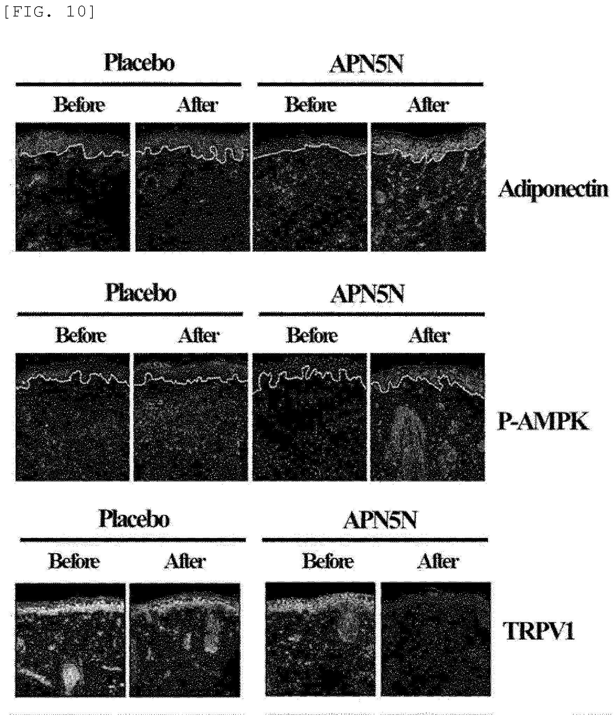

In order to classify patients with sensitive skin, a double-blind organ application study was executed by conducting the lactate puncture test (Contact Dermatitis 2010; 62: 137-49), which is the most widely used and known reliable test for diagnosis of sensitive skin (a total of 54 persons, the peptide of the present invention P5N: GLYYF-NH₂ (APN5N) 0.01% vs control (placebo), 27 persons per application group). From the volunteers who agreed to the biopsy (peptide P5N: GLYYF-NH₂ (APN5N) of the present invention 0.01% vs control material (placebo), 8 persons per application group), the skin tissues at the facial baseline and the skin tissues after 8 weeks application were obtained from total 4 sites (for 2 sites per each) by 2 mm punch. For the obtained skin tissues, quantitative PCR analysis and immunohistochemistry were performed for sensitive skin-related genes and markers including adiponectin. As a result of the lactate cut tests at the 2nd visit (4 weeks after application) and the 3rd visit (8 weeks after application), respectively, with classifying "the person who shows two or more irritant feels even once for 10 minutes as positive" in the lactic acid cut test, the sensitive skin of the test group to which APN5N was applied was significantly improved to non-sensitive skin (FIG. 9). Further, at the second visit, it could be seen that 29.6% of the test group was improved from the sensitive skin to the non-sensitive skin. On the other hand, the control showed 14.8% improvement. There was no significant difference between the above two groups (p=0.327, fisher's exact test). Further, at the 3rd visit, the initial sensitive skin improved to non-sensitive skin in 48.1% of the test group, whereas only 14.8% of the control improved to non-sensitive skin, which is the same as at the 2nd visit. Therefore, it showed a significant difference between the two groups (p=0.018). Further, since the expression of adiponectin was reduced in the patients having sensitive skin, a change in adiponectin gene expression after APN5N treatment could be an important indicator of sensitivity improvement. Therefore, as a result of confirming the expression of adiponectin, the sub-signaling factor P-AMPK of adiponectin and the sensitivity inducing factor TRPV1 (FIG. 10) through biopsy, the expression adiponectin and P-AMPK was increased after application in the test group containing APN5N, whereas the expression of TRPV1 was decreased. After application of the test substance containing APN5N twice a day for 8 weeks in the test group, the expression of adiponectin increased statistically and significantly more than 4 times compared to before application. On the other hand, there was no statistically significant change in the expression of adiponectin in the control after application of the control material.

### 9. Confirmation of hair growth promoting efficacy

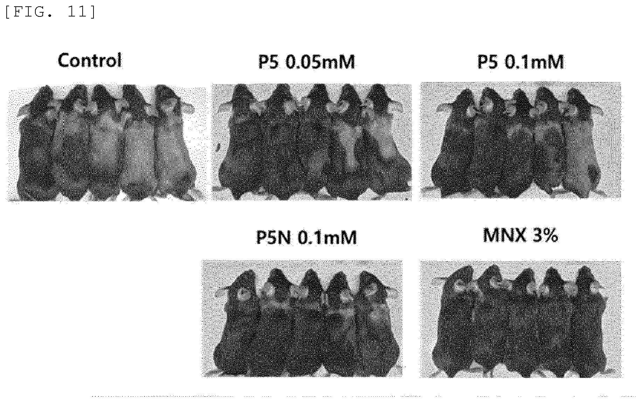

It is known that all the hairs of a mouse go through a process in which the hair enters the resting phase at 7 to 8 weeks of age and is converted back to the growing phase. Accordingly, the effect of the peptide of the present invention on the growth phase inducing process was confirmed using C57BL/6 mice. Specifically, 3% of minoxidil (MNX), which is commonly used as a treatment for hair loss and hair growth, on the skin of the lower back of the mouse, the existing adiponectin-derived peptide P5 (GLYYF, 0.05 mm and 0.1 mM), and the present peptide P5N (GLYYF-NH₂) were applied at 0.1 mM concentration, respectively, once a day. Thereafter, hair growth was investigated. As a result, in the group treated with the peptide of the present invention as in the positive controls, it was confirmed in the group treated with the peptide of the present invention as in the positive controls that, after about 4 weeks, a proportion of the region where hair was grown in the growth phase among the region where hair was first removed is increased (FIG. 11).

## Claims

1. A peptide comprising an amino acid sequence SEQ ID NO: 1 or 2, and having an amino group at C-terminus.

2. The peptide according to claim 1, wherein at least one amino acid is a D-form.

3. The peptide according to claim 1, wherein one amino acid is a D-form.

4. A peptide comprising an amino acid sequence SEQ ID NO: 1 or 2, wherein at least one amino acid is a D-form.

5. The peptide according to claim 4, wherein one amino acid is a D-form.

6. A cosmetic composition comprising the peptide according to any one of claims 1 to 5.

7. The cosmetic composition according to claim 6, wherein the composition is adapted to improve skin wrinkles, improve sensitive skin, improve dermatitis or promote hair growth.

8. A pharmaceutical composition for improving skin wrinkles, improving dermatitis or promoting hair growth, comprising the peptide according to any one of claims 1 to 5.

9. A pharmaceutical composition for treatment of metabolic diseases, comprising the peptide according to any one of claims 1 to 5.
